# EUROPEAN PATENT SPECIFICATION

(11) **EP 3 186 370 B1**
(45) Date of publication and mention of the grant of the patent: **01.01.2020**
(21) Application number: 15780935.1
(22) Date of filing: 27.08.2015
(51) Int. Cl.: C12N 9/14, C12N 9/36, A61K 38/16, A61K 38/46

(54) **ENZYME WITH HIGH LYTIC ACTIVITY AGAINST ENTEROCOCCUS CELL AND A METHOD OF MODIFICATION OF THE GENE THEREOF, ENABLING OVERPRODUCTION OF ACTIVE ENZYME IN BACTERIAL CELLS**
ENZYM MIT HOHER LYTISCHER AKTIVITÄT GEGEN ENTEROCOCCUS-ZELLEN UND VERFAHREN ZUR VERÄNDERUNG DES GENS DAVON ZUR ÜBERPRODUKTION VON AKTIVEM ENZYM IN BAKTERIENZELLEN
ENZYME À ACTIVITÉ LYTIQUE ÉLEVÉE CONTRE DES CELLULES D'ENTEROCOCCUS ET PROCÉDÉ DE MODIFICATION DU GÈNE ASSOCIÉ, PERMETTANT LA SURPRODUCTION D'ENZYMES ACTIVES DANS DES CELLULES BACTÉRIENNES

(30) Priority: 27.08.2014 PL 40928314
(43) Date of publication of application: 05.07.2017
(73) Proprietor: Instytut Biochemii I Biofizyki Polskiej Akademii Nauk, 02-106 Warszawa (PL); Instytut Immunologii i Terapii Doswiadczalnej Polskiej Akademii Nauk, 53-114 Wroclaw (PL)
(72) Inventor: LOBOCKA, Malgorzata, 02-798 Warszawa (PL); GOZDEK, Agnieszka, 00-714 Warszawa (PL); KOSAKOWSKI, Jaroslaw, 02-796 Warszawa (PL); RÓZYCKA, Aleksandra, 05-320 Mrozy (PL); WEBER-DABROWSKA, Beata, 51-628 Wroclaw (PL); GÓRSKI, Andrzej, 02-784 Warszawa (PL)
(74) Representative: Witek, Rafal
(86) International application number: PCT/IB2015/056510
(87) International publication number: WO 2016/030855

(56) References cited:
- EP-A1- 2 157 100

## Description

The object of the invention is an Ef12c29-ami lysin of an *Enterococcus faecalis* bacteriophage, having high lytic activity against *Enterococcus* cells, and a sequence of the gene encoding it, a method of modification of this gene and of a product thereof enabling overproduction of modified lysin in an active form in bacterial cells, and use of the modified lysin for peptidoglycan digestion and destruction of cells of *Enterococcus* or other bacteria, for the purposes of biochemical preparations in research, biotechnology and industry and as an antibacterial agent.

The main problem in controlling undesired bacteria and in obtaining contents of bacteria in biochemical preparations, biotechnology and industry is to cause destruction of bacterial cells.

A protective element of bacterial cells, which counteracts intracellular osmotic pressure, is a rigid cell wall built of peptidoglycan, surrounding the cytoplasm (Silhavy et al., 2010). It is separated from the cytoplasm by a cytoplasmic membrane. Cell wall damage leads to cell death and to release of its contents due to the so-called osmotic lysis - rupturing of the cytoplasmic membrane inflated by intracellular pressure. Therefore, numerous enzymes capable of damaging cell wall by digesting chemical bonds in peptidoglycan, including the commonly known lysozyme, have bactericidal activity. They are also used to obtain the contents of bacterial cells, e.g. during isolation of bacterial nucleic acids or proteins (Salazar and Asenjo, 2007).

The ability to digest peptidoglycan is exhibited *inter alia* by endolysins - bacteriophage-encoded lysins (Nelson et al., 2012). They are accumulated in cells during the phage lytic development. They digest the bonds between structural elements of the cell wall peptidoglycan, causing cell lysis and the release of the phages multiplied therein to the outside. The cell wall of Gram-positive bacteria is thick and in direct contact with the ambient environment. This enables external use of endolysins as agents killing these bacteria in antibacterial therapies and disinfection, as well as in biochemical preparation, as agents aiding the release of cell contents. Due to bacteriolytic activity and narrow specificity, often limited to a species, of Gram-positive bacteria bacteriophage lysins, they are also exceptionally safe tools in the struggle against multidrug-resistant strains of bacterial pathogens (Pastagia et al., 2013).

The specificity of phage endolysins and lysins of different origin is limited by the presence in these enzymes of domains with affinity to cell walls of specific bacteria or catalytic activity limited to specific bonds between peptidoglycan components (Nelson et al., 2012). These components may differ in bacteria from different taxons (Vollmer et al., 2008).

*Enterococcus faecalis* and *Enterococcus faecium* are Gram-positive lactic acid bacteria, forming a natural component of the microflora inhabiting mucous membranes, mainly of the human and animal gastrointestinal system. As typical representatives of Gram-positive cells, the enterococci have thick, multilayered peptidoglycan composed of polysaccharides, peptides and teichoic acid (Vollmer et al., 2008). However, the peptide bridge crosslinking the major structural elements of their peptidoglycan comprises an atypical D-asparagine residue.

Additionally, their peptidoglycan is characterized by high O-acetylation levels (Pfeffer et al., 2006). Therefore, lytic enzymes that effectively digest peptidoglycan of other bacteria, such as lysozyme, are inactive or show weak activity against enterococcal cell walls. Whereas a combination of lysozyme with mutanolysin is used for enterococcus cell lysis, effective lysis requires high concentrations of both enzymes and long reaction times (Billot-Klein et al., 1996).

This is a problem while using the commonly available preparations for rapid bacterial lysis against enterococcus cells or in nucleic acids or proteins isolation methods from microorganism consortia that may contain enterococci, e.g. for the purposes of metagenomic studies.

EP2157100 describes peptidoglycan lysing enzymes from different sources of bacteria including bacteria of genus *Enterococcus.* One of them is the enzyme digesting peptidoglycan (Fab20VL). Despite high similarity of amino acid sequence known enzyme possess significant different lytic activity than the phage Ef12c29 amidase.

Furthermore, in recent years, the *E. faecalis* and *E. faecium* strains have become one of the major causes of hospital-derived infections, due to acquiring drug resistance, including resistance against vancomycin considered the last-chance antibiotic (Arias and Murray, 2012).

Particularly in individuals with weakened immune response, enterococci may cause serious diseases: bacteremia, endocarditis or urinary tract inflammation. The number of patient-isolated *Enterococcus* strains resistant to beta lactam antibiotics and vancomycin, considered the last-chance antibiotic, is increasing at an alarming rate. In this respect, lytic enzymes, capable of effective destruction of living *Enterococcus* cells, are desired as antibacterial agents against enterococci, alternative to antibiotics.

The object of the present invention is to provide a lytic enzyme, capable of being overproduced in bacterial cells, enabling efficient destruction of cell walls of Gram-positive bacteria, in particular of the genus *Enterococcus,* and characterized by high enzymatic activity, particularly against the *Enterococcus* cell wall.

The subject of the invention is a polypeptide having the activity of digestion of peptidoglycan, particularly one produced by bacterial cells of the genus *Enterococcus,* comprising the amino acid sequence designated as SEQ ID NO: 3. Preferably, the polypeptide according to the invention comprises an amino acid sequence designated as SEQ ID NO: 4.

A further subject of the invention is a polynucleotide comprising a sequence encoding the polypeptide according to the invention as defined above. Preferably, the polynucleotide according to the invention comprises the nucleotide sequence designated as SEQ ID NO: 1.

A further subject of the invention is a use of the polypeptide according to the invention as defined above, for non-therapeutic digestion of peptidoglycan, particularly one produced by bacterial cells of the genus *Enterococcus.*

A further object of the invention is the polypeptide according to the invention as defined above, for use in the treatment or prevention of bacterial infections, particularly those caused by bacteria of the genus *Enterococcus.*

### Detailed description of the invention

The object of one of the demonstrated embodiments of the invention is a phage lysin having an amino acid sequence with features differentiating it from known lysins and with high lytic activity against bacteria, particularly including bacteria of the genus *Enterococcus.*

A further embodiment of the invention relates to a modification of the said lysin protein, providing for overproduction thereof in bacterial cells in an active form.

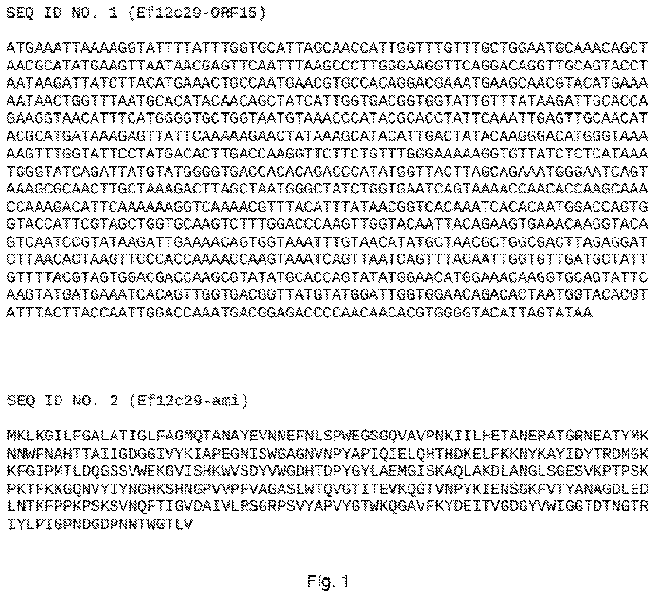

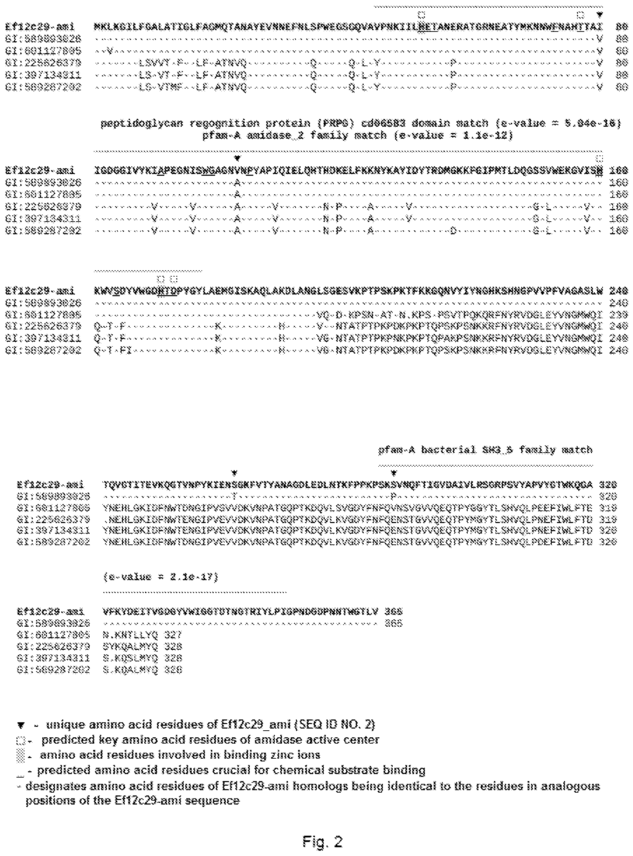

While searching for genes encoding potential endolysins in the genome of an *Enterococcus faecalis* bacteriophage Ef12c29, the inventors have identified the Ef12c29_ORF15 gene (SEQ ID NO: 1), wherein the protein product thereof being designated as Ef12c29-ami (having the amino acid sequence of SEQ ID NO: 2), has properties characteristic of lytic enzymes - N-acetylmuramoyl-L-alanine amidases (Fig. 1; Fig. 2). Unexpectedly it was found to differ substantially from homologs deposited in protein databases by the presence of at least four unique amino acid residues in the regions of the predicted active center and the predicted substrate-binding domain. Lytic activity of only one of the homologs (designated as GI:225626379 on Fig. 2), partially differing from Ef12c29-ami within the predicted catalytic portion and totally different within the predicted substrate-binding portion, has been initially verified previously (Son et al., 2010). The others were predicted on the basis of the amino acid sequence translated from the DNA sequence only. Interestingly, the four recently purified proteins having lytic activity against *Enterococcus* (Sugahara et al., 2007; Yoong et al., 2004; Zhang et al., 2013; Uchiyama et al., 2011) did not show any significant homology with Ef12c29-ami.

It was unexpectedly found that the attempts to clone the whole gene encoding the Ef12c29-ami protein were unsuccessful, probably due to lysis of the cells harboring the correct clones.

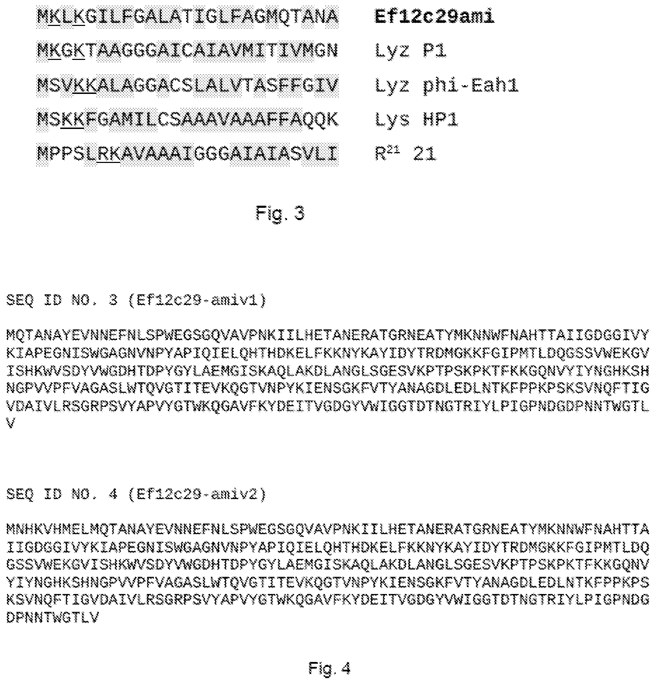

Although most of phage endolysins may be overproduced in bacterial cells, because their amino acids sequences do not contain signals for transport across the cytoplasmic membrane, there are such endolysins having a signal for trans-membrane transport at their N-terminus, the so-called SAR sequence (Young, 2005). Overproduction of these endolysins in bacterial cells is problematic. By penetrating through the cytoplasmic membrane they gain access to the cell wall, digest it and cause lysis. Until now, the so-called 'consensus' sequence for endolysin SAR regions has not been found. It is only known that they are hydrophobic regions having sequences that may resemble transmembrane domains. The analysis of the amino acid sequence of the Ef12c29-ami protein, performed for the purposes of the present invention, demonstrated that the N-terminus thereof comprises clusters of hydrophobic amino acid residues, similar to those present in the known SAR domains of phage endolysins and that it could potentially play the role of a transmembrane domain mediating in enzyme transport across the cytoplasmic membrane (Fig. 3).

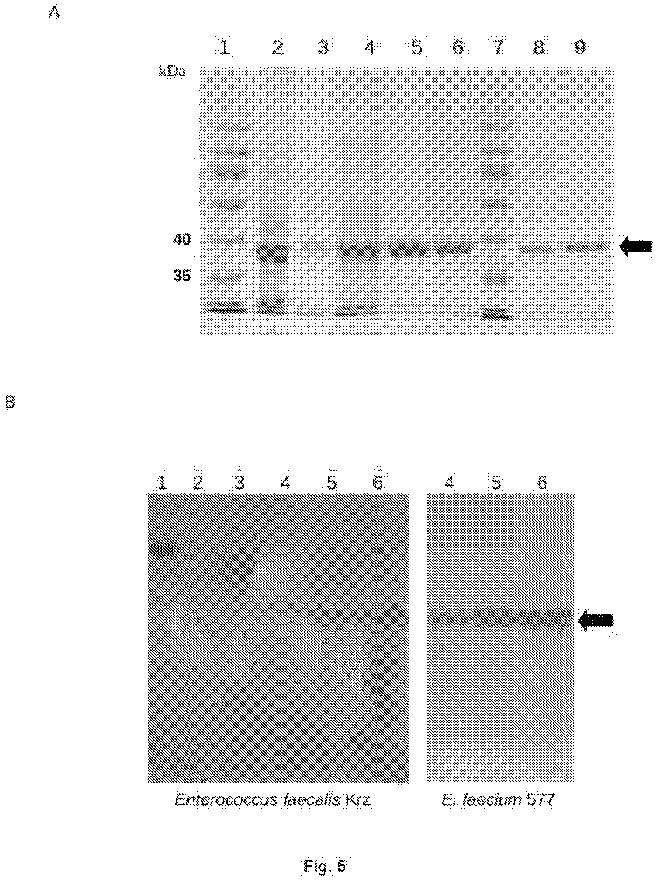

In order to eliminate the Ef12c29-ami endolysin toxicity to bacterial cells, it was attempted to clone the amplified gene encoding Ef12c29-ami, in a version deprived of a DNA fragment from the 5'-end of the gene encoding 19 amino acid residues from the N-terminus of the protein. The gene deprived of 5'-end, encoding a truncated version of the Ef12c29-ami protein, designated for the purposes of this invention as Ef12c29-amiv1 (SEQ ID NO: 3), was cloned to the pCOLDIII plasmid in such a way that the deleted fragment of the 5'-end would be replaced with the ATGAATCACAAAGTGCATATGGAGCTC sequence of the pCOLDIII vector, linked in frame to the truncated gene sequence for the Ef12c29-ami protein and encoding a fragment with the amino acid sequence of MNHKVHMEL. Transformants were obtained without any problems, harboring an insert with a desired sequence, which overproduced the Ef12c29-ami protein, having a sequence of 19 amino acid residues of the N-terminus replaced with the MNHKVHMEL sequence, designated for the purposes of the present invention as Ef12c29-amiv2 (SEQ ID NO: 4) (Fig. 4). The Ef12c29-amiv2 protein was obtained in a version comprising six histidine residues at the terminus, which allowed its purification using standard methods (Fig. 5).

The known SAR sequences play a key role in the process of activation of the endolysins comprising these (Young, 2014). Unexpectedly, during the work leading to the present invention, it was determined that the Ef12c29-amiv2 protein is lytically-active despite the lack of an original Ef12c29-ami protein N-terminal fragment therein.

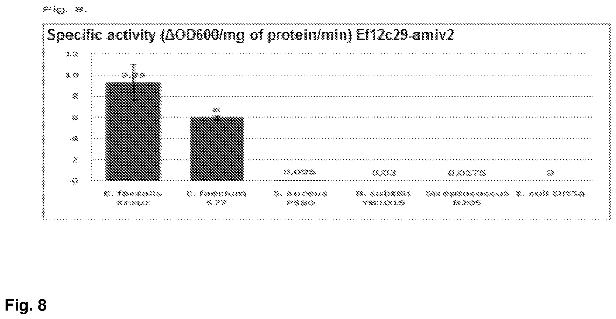

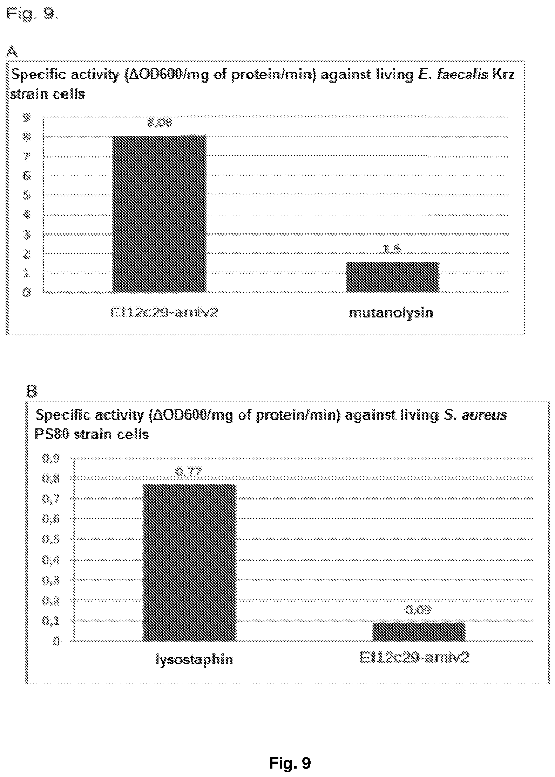

It was shown that the protein is active both in the lysis of dead and living bacterial cells of Gram-positive bacteria, including in particular bacterial cells of the genus *Enterococcus* (Fig. 5 - 8). Moreover, the specific activity of Ef12c29-amiv2, measured as the decrease in optical density of the bacterial suspension of the *Enterococcus* genus over time, per mg of protein, proved to be several-fold higher than the specific activity of the commercially available and utilized so far for the lysis of *Enterococcus* cells mutanolysin of *Streptomyces globisporus* (Sigma-Aldrich) measured in parallel experiments (Fig. 9). The one measured with *Enterococcus* cells as a substrate proved to be higher from the specific activity of lysostaphin as well, measured with the substrate of this enzyme - *Staphylococcus aureus* cells. It was also established that the Ef12c29-amiv2 lysin is active in the lysis of living cells of Gram-positive bacteria different than bacteria of the genus *Enterococcus,* such as *Staphylococcus aureus, Bacillus subtilis* and *Streptococcus,* although the activity thereof was in this case weaker than the activity with the optimal substrate - which proved to be the cells of the genus *Enterococcus* (Fig. 8). This does not preclude modifications of Ef12c29-amiv2 that alter specificity thereof. It is therefore clear that Ef12c29-amiv2 may be successfully used to lyse bacterial cells or to destroy isolated peptidoglycan thereof, with all possible applications, including obtaining of the contents of bacteria in processes of isolation of cellular components thereof and as an anti-bacterial agent in disinfection of materials and environment, as well as in prevention and treatment of infections. Optimal functioning of the enzyme in physiological conditions (pH 7.0, 37°C) makes it particularly useful in medical and veterinary applications, through administration thereof into the organism.

The embodiments of the invention are illustrated by the following figures:
Figure 1 shows the nucleotide sequence of the Ef12c29 phage endolysin gene, designated as Ef12c29_ORF15 (SEQ ID NO: 1) and the amino acid sequence of the product of this gene, designated as Ef12c29-ami (SEQ ID NO: 2), according to the present invention.
Figure 2 shows an alignment of the Ef12c29-ami endolysin amino acid sequence with sequences of homologous endolysins from protein database, with indication of amino acid residues unique to the Ef12c29-ami endolysin (indicated by the ▼ mark). The numbers of Ef12c29-ami homologs from the NCBI protein database indicate the following: GI:589893026 - lysin IME-IF4_42 of Enterococcus phage IME-IF4 (predicted on the basis of DNA sequence: NC_023551.1); GI:601127805 - N-acetylmuramoyl-L-alanine amidase family protein of Enterococcus phage AUEF3 (predicted on the basis of DNA sequence: AHN83275); GI:225626379 - EFAP1_gp02 amidase of Enterococcus faecalis phage EFAP-1; GI:397134311 - putative endolysin of Enterococcus phage EfaCPT1 (predicted on the basis of DNA sequence: JX193904); GI:589287202 - lysin, N-acetylmuramoyl-L-alanine amidase [Enterococcus phage IME_EF3] (predicted on the basis of DNA sequence: NC_023595.2).
Figure 3 shows an alignment of the amino acid sequence of the N-terminus of the Ef12c29-ami protein with sequences of N-termini of selected phage endolysins comprising the SAR domain. Hydrophobic amino acid residues are highlighted in grey, positively charged residues are underlined. N-termini of the known endolysins with the SAR domain are shown according to Young (2005).
Figure 4 shows the amino acid sequences of the Ef12c29-ami lysin, modified by removing a 19 amino acid residues long fragment thereof from the N-terminus of the protein and designated as Ef12c29-amiv1 (SEQ ID NO: 3), and the amino acid sequence of the Ef12c29-ami lysin modified by replacing 19 amino acid residues from the N-terminus thereof with a fragment with the sequence of MNHKVHMEL and designated as Ef12c29-amiv2 (SEQ ID NO: 4), according to the present invention.
Figure 5 shows the consecutive steps of purification of the Ef12c29-amiv2 protein and preliminary verification of the Ef12c29-amiv2 protein lytic activity using a zymogram method. The proteins were separated by electrophoresis in a polyacrylamide gel with SDS. The consecutive lanes on the gel (A) represent the obtained after separation in polyacrylamide gel: 1 and 7 - size marker proteins (PageRuler Prestained Protein Ladder, Thermo Scientific, Cat. No. 26616; the size of selected marker proteins given in kDa), 2 - proteins from pellet obtained after lysis and centrifugation of the BL21(DE3)-T1R strain cells, harboring a derivative of the pCOLDIII plasmid, encoding the Ef12c29-amiv2 protein in non-denaturing conditions, 3 - lysate proteins dissolved in buffer with 8 M urea, 4 - lysate proteins dissolved in buffer with 8 M urea remaining in the filtrate from the cobalt substrate TALON column (FT), 5 - proteins of the first eluate fraction from the column (E1), 6 - proteins of the second eluate fraction from the column (E2), 8 - proteins of the E1 fraction after dialysis, proteins of the E2 fraction after dialysis. Ef12c29-amiv2-6xHis is indicated by arrow. Preliminary detection of lytic activity of the purified Ef12c29-amiv2 protein was conducted by a zymogram method (B), after protein separation in polyacrylamide gel containing 0.2% suspension of killed Enterococcus faecalis Krz (left panel) or Enterococcus faecium 577 (right panel) cells. The consecutive lanes on gel B represent: 1 - size marker proteins, 2 - proteins of cell lysate BL21/pCOLDIII-Ef12c29-amiv2 dissolved in buffer with 8 M urea, 3 - lysate proteins dissolved in buffer with 8 M urea remaining in the filtrate from the cobalt substrate TALON column (FT), 4 - proteins of the first eluate fraction from the column (E1) in 8 M urea, 5 and 6 - proteins of the E1 fraction after dialysis. Clear zones in the gel indicating zones of cell lysis are marked by arrow.

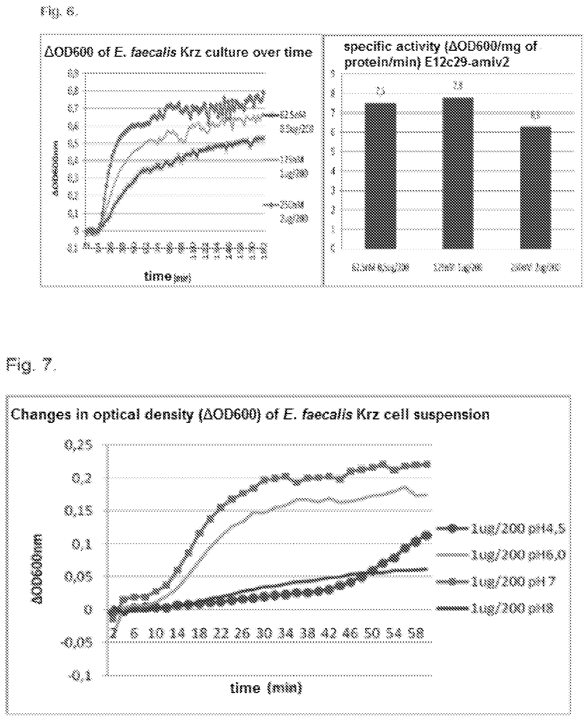

Figure 6 shows a graph of dependency of Ef12c29-amiv2 endolysin lytic activity against the Enterococcus faecalis Krz strain cells on the endolysin protein concentration in the reaction mixture. Enzyme processivity is shown as the decrease in optical density of the bacterial suspension over time (left panel) and the specific activity of the enzyme calculated on this basis as the decrease in optical density of the bacterial suspension per minute per mg of the purified Ef12c29-amiv2 protein.
Figure 7 shows a graph of dependency of Ef12c29-amiv2 endolysin lytic activity on the pH of the reaction mixture. Activity measurements were conducted in Bioscreen apparatus (see example 4) following the addition of 1 µg of the purified Ef12c29-amiv2 protein to 200 µl of cell suspension. Enzyme activity is shown as the decrease in optical density of the bacterial suspension over time. against
Figure 8 shows a comparison of Ef12c29-amiv2 endolysin specific activity against Enterococus faecalis Krz, Enterococcus faecium 577, Staphylococcus aureus PS80, Bacillus subtilis YB1015, Streptococcus B205 and Escherichia coli DH5α cells.
Figure 9 shows a comparison of Ef12c29-amiv2 specific lytic activity with lysostaphin and mutanolysin.

In order to better understand the essence of the invention it is illustrated by the examples below, showing the method of cloning of the modified Ef12c29-ami endolysin gene, the method of overexpression of this gene and of purification of the product thereof, and the determination of lytic activity and specificity of the modified Ef12c29-ami2 endolysin. They also demonstrate a significantly higher activity of the modified Ef12c29-ami2 endolysin in digesting cell walls of the *Enterococcus* cells in comparison with mutanolysin. In all examples, unless stated otherwise, standard molecular biology methods described by Sambrook et al. (1989) were used.

Example 1. Identification of a gene (in accordance with the sequence of SEQ ID NO: 1) encoding the active Ef12c29-ami endolysin (in accordance with the sequence of SEQ ID NO: 2) in the *Enterococcus faecalis* bacteriophage Ef12c29 genome.

As a result of bioinformatical analysis of the Ef12c29 bacteriophage genome sequence, a gene was identified (SEQ ID NO: 1) encoding a protein designated according to the present invention Ef12c29--mi (SEQ ID NO. 2) (Fig. 1) having features of a potential endolysin showing amidase activity (Fig. 2). In the regions of the potentially critical Ef12c29-ami domains responsible for catalytic activity or substrate binding, four unique amino acid residues were detected, differentiating Ef12c29-ami from the sequence of the closest protein homolog from the NCBI database, predicted on the basis of translation of a DNA sequence deposited in the GenBank database. Additionally, at the N-terminus of the Ef12c29-ami protein a highly hydrophobic domain having features of the SAR domain was detected, similar to a transmembrane domain (Fig. 3). The gene encoding the full-length Ef12c29-ami protein was amplified with primers 5'-CACCATGAAATTAAAAGGTATTTTATTT and 5'-TACTAATGTACCCCACGTGTTGT, the amplification product was ligated with linearized DNA of the pBAD TOPO vector from Invitrogen. Competent cells of the *Escherichia coli* GC5™ (F- F80IacZDM15 Δ[IacZYAargF]U169 endA1 recA1relA1 gyrA96 hsdR17 [rk-,mk+] phoA supE44 thi-1 I-T1R) were transformed with the ligation mixture. The transformants were seeded on a complete medium with ampicillin, glucose and without arabinose, in the previously described selection conditions for the pBAD-TOPO plasmid, ensuring maximal repression of the cloned gene transcription from the pBAD promoter controlling it (Guzman et al., 1995). Despite obtaining numerous transformant colonies, it was unexpectedly found that the cells thereof undergo lysis on the plate, which renders plasmid DNA isolation impossible. After seeding them to liquid cultures, no growth was observed due to lysis. Cells from few cultures that had not undergone lysis did not contain the plasmid. The obtained result indicates high lytic activity of the Ef12c29-ami protein against bacterial peptidoglycan and the ability of this protein to penetrate from cytoplasm to periplasm in the absence of helper proteins, which is consistent with the presence of a potential signal for trans-membrane transport at the N-terminus of Ef12c29-ami.

The attempt to clone the Ef12c29-ami encoding gene was repeated in the pCOLDIII vector (Quing et al., 2004). DNA fragments obtained as a result of amplification of the whole Ef12c29-ami encoding sequence with the following primers
5'-TAAGAGCTCATGAAATTAAAAGGTATTTTATTTG and
5'-ACATCTAGATTAATGATGATGATGATGATGTACTAATGTACCCCACGTGT,
were digested with Sacl and Xbal restriction enzymes and inserted by ligation at the Sacl-Xbal fragment site of the pCOLDIII plasmid polylinker. The thus obtained amplicons contained the full-length Ef12c29-ami endolysin gene encoding the protein with the addition of a six-histidine marker at the C-terminus and the MNHKVHMEL sequence at the N-terminus. The ligation mixture was then transformed into *Escherichia coli* DH5α (F- ϕ80IacZΔM15 Δ[IacZYA-argF] U169 recA1 endA1 hsdR17[rk-, mk+] gal-phoA supE44 λ- thi-1 gyrA96 relA1) strain cells. The transformants were selected on complete medium with ampicillin selective for the plasmid. Despite several repetitions of the experiment only few transformant clones were obtained. They carried inserts of the amplified Ef12c29-ami gene but all the inserts harbored mutations in the gene sequence causing early termination of translation, hence did not encode a functional Ef12c29-ami protein. The obtained results unambiguously indicate the toxicity of the Ef12c29-ami endolysin produced in *Escherichia coli* cells from the full-length gene cloned in the pCOLDIII plasmid, consistent with the previously observed lysis of cells harboring the complete gene for the protein.

Example 2. Construction of a modified Ef12c29-ami endolysin gene, encoding a modified Ef12c29-amiv2 protein (in accordance with the sequence of SEQ ID NO: 4) not exhibiting toxicity to bacterial cells producing it.

In order to obtain a modified form of the Ef12c29-ami endolysin, capable of being overproduced in bacterial cells, the Ef12c29-ami gene of the Ef12c29 phage deprived of a 57 base pair fragment from the 5'-end, encoding a potential signal for trans-membrane transport, was amplified with the following primers:
5'-TTGAGCTCATGCAAACAGCTAACGCATATGAAGTTA and
5'-ACATCTAGATTAATGATGATGATGATGATGTACTAATGTACCCCACGTGT,
so that the amplification product encoded a truncated form of Ef12c29-ami designated in the present invention as Ef12c29-amiv1 (in accordance with the sequence of SEQ ID NO: 3), with the addition of a six-histidine marker. DNA fragments obtained as a result of amplification were digested with Sacl and Xbal restriction enzymes and inserted by ligation at the Sacl-Xbal fragment site of the pCOLDIII plasmid. The ligation mixture was transformed into competent cells of the DH5α strain. Numerous transformants were obtained on complete medium with ampicillin. Plasmids isolated therefrom contained inserts having an unchanged sequence of the amplified Ef12c29-ami gene fragment, so that pCOLDIII plasmid derivative with the insert encoded the Ef12c29-amiv1 protein with the addition of the MNHKVHMEL sequence at the N-terminus and designated in the present invention as Ef12c29-amiv2 (in accordance with the sequence of SEQ ID NO: 4). One of the obtained plasmids with the correct insert sequence was used for further studies. It was transferred using a transformation method to the *E. coli* BL21(DE3)-T1^{R} (F-ompT hsdSB[rB-mB-] gal dcm I[DE3] tonA) strain cells. In cell culture of the obtained transformant, expression of the cloned gene was induced according to the standard procedure (Qing et al., 2004). After induction, a protein was observed among the proteins of this strain, with molecular mass corresponding to the predicted molecular mass of the Ef12c29-amiv2 protein (∼40 kDa). High intracellular levels of the Ef12c29-amiv2 protein indicated lack of Ef12c29-amiv2 toxicity to bacterial cells producing the protein.

Example 3. Preliminary evaluation of lytic activity of the Ef12c29-amiv2 protein against cell walls of killed cells of the *Enterococcus* genus.

400 ml of fresh LB medium with ampicillin was seeded with 1/100 volume of *E. coli* BL21(DE3)-T1^{R} cell culture, comprising the pCOLDIII-Ef12c29-amiv2 plasmid and sampled from an overnight culture in the same medium. The culture was conducted in 37°C until reaching cell optical density at wavelength of 600 nm (OD₆₀₀) = 0.7-1.0. Next, expression of the Ef12c29-amiv2 encoding gene was induced by transferring the culture to temperature of 16°C and adding 1 mM IPTG. After 16 hours the culture was centrifuged to obtain bacterial pellet. For complete recovery of Ef12c29-amiv2 the protein was purified in denaturing conditions. Bacteria were lysed in buffer containing 20 mM Tris-HCI pH 7.0 and 500 mM NaCl, by sonication, the obtained lysate was centrifuged and the obtained pellet was dissolved in binding buffer containing 200 mM sodium phosphate pH 7.8; 500 mM NaCl, 8 M urea. The dissolved proteins were bound on the TALON Metal Affinity Resin substrate (Clontech) by mixing overnight in 4°C, using 1 ml of substrate per 10 ml of protein dissolved in the binding buffer. Washing the substrate with bound proteins and elution were performed using buffers with lowered pH (pH= 6 and 4, respectively). Protein solutions obtained after elution were sealed in dialysis sacks (Sigma, permeability 14 000 Da) and dialyzed sequentially for 2-12 hours on a magnetic stirrer in buffers (0.5 M NaCl, 50 mM KiPO₄ pH 7.8; 0.4 M arginine) containing decreasing concentrations of urea (in sequence 4 M, 2 M, 1 M, 0.5 M) and finally in buffer without urea. From 400 ml of culture of induced cells 5 mg of purified Ef12c29-amiv2 protein were routinely obtained. The purified enzyme was stored in aliquots in -80°C in buffer containing 0.5 M NaCl, 50 mM KiPO₄ pH 7.8; 0.4 M arginine, 50% glycerol and after thawing was used for activity measurements. Proteins obtained at consecutive Ef12c29-amiv2 purification stages and the preliminary evaluation of Ef12c29-amiv2 lytic activity is shown on Fig. 5. Ef12c29-amiv2 lytic activity was roughly estimated using a zymogram method, according to the known procedure (Leclerc and Asselin, 1989). Polyacrylamide gel (10%) wherein the proteins were separated to estimate lytic activity, comprised 0.2% volume of autoclaved bacterial cells of the *Enterococcus faecalis* Krz strain or *Enterococcus faecium,* from the pellet of an overnight culture which was centrifuged after autoclaving.

Example 4. Evaluation of Ef12c29-amiv2 lytic activity against living cells of the *Enterococcus faecalis* Krz strain and determination of optimal pH for enzyme activity.

Ef12c29-amiv2 lytic activity against living *Enterococcus faecalis* cells and the effect of various factors on this activity were determined using the Bioscreen C MBR apparatus (Growth Curves USA), in 100-well plates with wells for 200 µl of bacterial culture, in 37°C with shaking. Living bacterial cells used in the assays were derived from an exponentially growing culture in LB medium. After the culture has reached optical density OD₆₀₀=0.4 it was centrifuged and the pellet was suspended in STB buffer: 150 mM NaCl, 10 mM Tris pH 4.5-8.8, so that the optical density (OD₆₀₀) of the suspension was 1. The suspension (190 µl) was added to the wells of the Bioscreen apparatus plate, and then the purified enzyme solution (10 µl) was added to the indicated amount and the decrease in optical density of the culture over time was measured. The purified enzyme was derived from thawed samples stored in -80°C in buffer containing 0.5 M NaCl, 50 mM KiPO₄ pH 7.8; 0.4 M arginine and 50% glycerol. The Ef12c29-amiv2 protein was found to be lytically-active against living cells of the *E. faecalis* Krz strain and the enzyme concentration at which it was characterized by the highest processivity in cell lysis was determined to be 125 nM (Fig. 6). After adding the enzyme to the cell suspension, a clear decrease in culture density was observed after only 14 minutes. By testing the effect of different pH values on the enzyme lytic activity it was established that the Ef12c29-amiv2 protein is the most active in pH 7 conditions, while in pH 6 conditions it shows an only slightly reduced lytic activity (Fig. 7).

Example 5. Evaluation of the range of Ef12c29-amiv2 lytic activity against living cells of Gram-positive bacterial strains representing different genera.

The range of Ef12c29 lytic activity against living cells representing strains of the selected Gram-positive bacterial species was determined using cells of the following strains: *E. faecium* 577, S. *aureus* PS80, *B. subtilis* YB1015 and *Streptococcus* B205. The *E*. *faecalis* Krz strain was used as positive control and the *E*. *coli* DH5α strain, representing Gram-negative bacteria, as negative control. The cell wall of Gram-negative bacteria cells is surrounded by an outer cell membrane, protecting them against lytic activity of externally administered endolysins. The measurements of Ef12c29-amiv2 lytic activity were done in the Bioscreen C apparatus, in accordance with example 4. The specific Ef12c29-amiv2 activity was calculated as the decrease in optical density of bacterial suspension (OD₆₀₀) per minute per mg of the Ef12c29-amiv2 protein. Unexpectedly, the Ef12c29-amiv2 protein showed high lytic activity in the assays, both against the *E. faecalis* Krz strain cells and *E. faecium* 577, wherein activity in the case of *E*. *faecium* 577 was only slightly lower (Fig. 8). Ef12c29-amiv2 lytic activity was observed also in the case of *S*. *aureus, B. subtilis* and *Streptococcus* cells but it was several fold lower.

Example 6. Comparison of the Ef12c29-amiv2 lysin specific activity with the specific activity of mutanolysin and lysostaphin.

In order to compare the Ef12c29-amiv2 lysin lytic activity with mutanolysin, often utilized in combination with lysozyme for lysis of *Enterococcus* cells, dynamics of the decrease in optical density of living *Enterococcus faecalis* Krz cells after the addition of the Ef12c29-amiv2 lysin or mutanolysin (Sigma-Aldrich) was studied in the Bioscreen C apparatus (see Example 4) (Fig. 9). The specific Ef12c29-amiv2 activity, measured as the decrease in cell optical density per minute per mg of protein of the added enzyme, proved to be several fold higher than mutanolysin specific activity. In a similar experiment, the specific activity of Ef12c29-amiv2 and lysostaphin were compared against living *Staphylococcus aureus* cells as the substrate. Although in this case the Ef12c29-amiv2 lysin showed an 8-fold lower lytic activity than lysostaphin, the comparison of the specific activities of Ef12c29-amiv2 and lysostaphin with substrates specific to each of the proteins (E. faecalis Krz and S. aureus PS80, respectively) proved to be in favor for the Ef12c29-amiv2 lysin.

### Sequence listing

<110> IBB PAN
   IITD PAN
<120> Polypeptide, nucleotide encoding it and use thereof
   <130> PK/2685/RW
   <160> 12
   <170> PatentIn version 3.5
<210> 1
   <211> 1098
   <212> DNA
   <213> Bacteriophage Enterococcus faecalis Ef12c29 Ef12c29_ORF15 gene encoding Ef12c29-ami protein
<400> 1
   atgaaattaa aaggtatttt atttggtgca ttagcaacca ttggtttgtt tgctggaatg 60
   caaacagcta acgcatatga agttaataac gagttcaatt taagcccttg ggaaggttca 120
   ggacaggttg cagtacctaa taagattatc ttacatgaaa ctgccaatga acgtgccaca 180
   ggacgaaatg aagcaacgta catgaaaaat aactggttta atgcacatac aacagctatc 240
   attggtgacg gtggtattgt ttataagatt gcaccagaag gtaacatttc atggggtgct 300
   ggtaatgtaa acccatacgc acctattcaa attgagttgc aacatacgca tgataaagag 360
   ttattcaaaa agaactataa agcatacatt gactatacaa gggacatggg taaaaagttt 420
   ggtattccta tgacacttga ccaaggttct tctgtttggg aaaaaggtgt tatctctcat 480
   aaatgggtat cagattatgt atggggtgac cacacagacc catatggtta cttagcagaa 540
   atgggaatca gtaaagcgca acttgctaaa gacttagcta atgggctatc tggtgaatca 600
   gtaaaaccaa caccaagcaa accaaagaca ttcaaaaaag gtcaaaacgt ttacatttat 660
   aacggtcaca aatcacacaa tggaccagtg gtaccattcg tagctggtgc aagtctttgg 720
   acccaagttg gtacaattac agaagtgaaa caaggtacag tcaatccgta taagattgaa 780
   aacagtggta aatttgtaac atatgctaac gctggcgact tagaggatct taacactaag 840
   ttcccaccaa aaccaagtaa atcagttaat cagtttacaa ttggtgttga tgctattgtt 900
   ttacgtagtg gacgaccaag cgtatatgca ccagtatatg gaacatggaa acaaggtgca 960
   gtattcaagt atgatgaaat cacagttggt gacggttatg tatggattgg tggaacagac 1020
   actaatggta cacgtattta cttaccaatt ggaccaaatg acggagaccc caacaacacg 1080
   tggggtacat tagtataa 1098
<210> 2
   <211> 365
   <212> PRT
   <213> Bacteriophage Enterococcus faecalis Ef12c29-ami
<400> 2
   Met Lys Leu Lys Gly Ile Leu Phe Gly Ala Leu Ala Thr Ile Gly Leu
   1               5                   10                  15
   Phe Ala Gly Met Gln Thr Ala Asn Ala Tyr Glu Val Asn Asn Glu Phe
               20                  25                  30
   Asn Leu Ser Pro Trp Glu Gly Ser Gly Gln Val Ala Val Pro Asn Lys
           35                  40                  45
   Ile Ile Leu His Glu Thr Ala Asn Glu Arg Ala Thr Gly Arg Asn Glu
       50                  55                  60
   Ala Thr Tyr Met Lys Asn Asn Trp Phe Asn Ala His Thr Thr Ala Ile
   65                  70                  75                  80
   Ile Gly Asp Gly Gly Ile Val Tyr Lys Ile Ala Pro Glu Gly Asn Ile
                   85                  90                  95
   Ser Trp Gly Ala Gly Asn Val Asn Pro Tyr Ala Pro Ile Gln Ile Glu
               100                 105                 110
   Leu Gln His Thr His Asp Lys Glu Leu Phe Lys Lys Asn Tyr Lys Ala
           115                 120                 125
   Tyr Ile Asp Tyr Thr Arg Asp Met Gly Lys Lys Phe Gly Ile Pro Met
       130                 135                 140
   Thr Leu Asp Gln Gly Ser Ser Val Trp Glu Lys Gly Val Ile Ser His
   145                 150                 155                 160
   Lys Trp Val Ser Asp Tyr Val Trp Gly Asp His Thr Asp Pro Tyr Gly
                   165                 170                 175
   Tyr Leu Ala Glu Met Gly Ile Ser Lys Ala Gln Leu Ala Lys Asp Leu
               180                 185                 190
   Ala Asn Gly Leu Ser Gly Glu Ser Val Lys Pro Thr Pro Ser Lys Pro
           195                 200                 205
   Lys Thr Phe Lys Lys Gly Gln Asn Val Tyr Ile Tyr Asn Gly His Lys
       210                 215                 220
   Ser His Asn Gly Pro Val Val Pro Phe Val Ala Gly Ala Ser Leu Trp
   225                 230                 235                 240
   Thr Gln Val Gly Thr Ile Thr Glu Val Lys Gln Gly Thr Val Asn Pro
                   245                 250                 255
   Tyr Lys Ile Glu Asn Ser Gly Lys Phe Val Thr Tyr Ala Asn Ala Gly
               260                 265                 270
   Asp Leu Glu Asp Leu Asn Thr Lys Phe Pro Pro Lys Pro Ser Lys Ser
           275                 280                 285
   Val Asn Gln Phe Thr Ile Gly Val Asp Ala Ile Val Leu Arg Ser Gly
       290                 295                 300
   Arg Pro Ser Val Tyr Ala Pro Val Tyr Gly Thr Trp Lys Gln Gly Ala
   305                 310                 315                 320
   Val Phe Lys Tyr Asp Glu Ile Thr Val Gly Asp Gly Tyr Val Trp Ile
                   325                 330                 335
   Gly Gly Thr Asp Thr Asn Gly Thr Arg Ile Tyr Leu Pro Ile Gly Pro
               340                 345                 350
   Asn Asp Gly Asp Pro Asn Asn Thr Trp Gly Thr Leu Val
           355                 360                 365
<210> 3
   <211> 346
   <212> PRT
   <213> artificial
   <220>
   <223> protein Ef12c29-amiv1
<400> 3
   Met Gln Thr Ala Asn Ala Tyr Glu Val Asn Asn Glu Phe Asn Leu Ser
   1               5                   10                  15
   Pro Trp Glu Gly Ser Gly Gln Val Ala Val Pro Asn Lys Ile Ile Leu
               20                  25                  30
   His Glu Thr Ala Asn Glu Arg Ala Thr Gly Arg Asn Glu Ala Thr Tyr
           35                  40                  45
   Met Lys Asn Asn Trp Phe Asn Ala His Thr Thr Ala Ile Ile Gly Asp
       50                  55                  60
   Gly Gly Ile Val Tyr Lys Ile Ala Pro Glu Gly Asn Ile Ser Trp Gly
   65                  70                  75                  80
   Ala Gly Asn Val Asn Pro Tyr Ala Pro Ile Gln Ile Glu Leu Gln His
                   85                  90                  95
   Thr His Asp Lys Glu Leu Phe Lys Lys Asn Tyr Lys Ala Tyr Ile Asp
               100                 105                 110
   Tyr Thr Arg Asp Met Gly Lys Lys Phe Gly Ile Pro Met Thr Leu Asp
           115                 120                 125
   Gln Gly Ser Ser Val Trp Glu Lys Gly Val Ile Ser His Lys Trp Val
       130                 135                 140
   Ser Asp Tyr Val Trp Gly Asp His Thr Asp Pro Tyr Gly Tyr Leu Ala
   145                 150                 155                 160
   Glu Met Gly Ile Ser Lys Ala Gln Leu Ala Lys Asp Leu Ala Asn Gly
                   165                 170                 175
   Leu Ser Gly Glu Ser Val Lys Pro Thr Pro Ser Lys Pro Lys Thr Phe
               180                 185                 190
   Lys Lys Gly Gln Asn Val Tyr Ile Tyr Asn Gly His Lys Ser His Asn
           195                 200                 205
   Gly Pro Val Val Pro Phe Val Ala Gly Ala Ser Leu Trp Thr Gln Val
       210                 215                 220
   Gly Thr Ile Thr Glu Val Lys Gln Gly Thr Val Asn Pro Tyr Lys Ile
   225                 230                 235                 240
   Glu Asn Ser Gly Lys Phe Val Thr Tyr Ala Asn Ala Gly Asp Leu Glu
                   245                 250                 255
   Asp Leu Asn Thr Lys Phe Pro Pro Lys Pro Ser Lys Ser Val Asn Gln
               260                 265                 270
   Phe Thr Ile Gly Val Asp Ala Ile Val Leu Arg Ser Gly Arg Pro Ser
           275                 280                 285
   Val Tyr Ala Pro Val Tyr Gly Thr Trp Lys Gln Gly Ala Val Phe Lys
       290                 295                 300
   Tyr Asp Glu Ile Thr Val Gly Asp Gly Tyr Val Trp Ile Gly Gly Thr
   305                 310                 315                 320
   Asp Thr Asn Gly Thr Arg Ile Tyr Leu Pro Ile Gly Pro Asn Asp Gly
                   325                 330                 335
   Asp Pro Asn Asn Thr Trp Gly Thr Leu Val
               340                 345
<210> 4
   <211> 355
   <212> PRT
   <213> artificial
   <220>
   <223> protein Ef12c29-amiv2
<400> 4
   Met Asn His Lys Val His Met Glu Leu Met Gln Thr Ala Asn Ala Tyr
   1               5                   10                  15
   Glu Val Asn Asn Glu Phe Asn Leu Ser Pro Trp Glu Gly Ser Gly Gln
               20                  25                  30
   Val Ala Val Pro Asn Lys Ile Ile Leu His Glu Thr Ala Asn Glu Arg
           35                  40                  45
   Ala Thr Gly Arg Asn Glu Ala Thr Tyr Met Lys Asn Asn Trp Phe Asn
       50                  55                  60
   Ala His Thr Thr Ala Ile Ile Gly Asp Gly Gly Ile Val Tyr Lys Ile
   65                  70                  75                  80
   Ala Pro Glu Gly Asn Ile Ser Trp Gly Ala Gly Asn Val Asn Pro Tyr
                   85                  90                  95
   Ala Pro Ile Gln Ile Glu Leu Gln His Thr His Asp Lys Glu Leu Phe
               100                 105                 110
   Lys Lys Asn Tyr Lys Ala Tyr Ile Asp Tyr Thr Arg Asp Met Gly Lys
           115                 120                 125
   Lys Phe Gly Ile Pro Met Thr Leu Asp Gln Gly Ser Ser Val Trp Glu
       130                 135                 140
   Lys Gly Val Ile Ser His Lys Trp Val Ser Asp Tyr Val Trp Gly Asp
   145                 150                 155                 160
   His Thr Asp Pro Tyr Gly Tyr Leu Ala Glu Met Gly Ile Ser Lys Ala
                   165                 170                 175
   Gln Leu Ala Lys Asp Leu Ala Asn Gly Leu Ser Gly Glu Ser Val Lys
               180                 185                 190
   Pro Thr Pro Ser Lys Pro Lys Thr Phe Lys Lys Gly Gln Asn Val Tyr
           195                 200                 205
   Ile Tyr Asn Gly His Lys Ser His Asn Gly Pro Val Val Pro Phe Val
       210                 215                 220
   Ala Gly Ala Ser Leu Trp Thr Gln Val Gly Thr Ile Thr Glu Val Lys
   225                 230                 235                 240
   Gln Gly Thr Val Asn Pro Tyr Lys Ile Glu Asn Ser Gly Lys Phe Val
                   245                 250                 255
   Thr Tyr Ala Asn Ala Gly Asp Leu Glu Asp Leu Asn Thr Lys Phe Pro
               260                 265                 270
   Pro Lys Pro Ser Lys Ser Val Asn Gln Phe Thr Ile Gly Val Asp Ala
           275                 280                 285
   Ile Val Leu Arg Ser Gly Arg Pro Ser Val Tyr Ala Pro Val Tyr Gly
       290                 295                 300
   Thr Trp Lys Gln Gly Ala Val Phe Lys Tyr Asp Glu Ile Thr Val Gly
   305                 310                 315                 320
   Asp Gly Tyr Val Trp Ile Gly Gly Thr Asp Thr Asn Gly Thr Arg Ile
                   325                 330                 335
   Tyr Leu Pro Ile Gly Pro Asn Asp Gly Asp Pro Asn Asn Thr Trp Gly
               340                 345                 350
   Thr Leu Val
           355
<210> 5
   <211> 27
   <212> DNA
   <213> artificial
   <220>
   <223> alternative 5' sequence
<400> 5
   atgaatcaca aagtgcatat ggagctc
   27
<210> 6
   <211> 9
   <212> PRT
   <213> artificial
   <220>
   <223> alternative N-sequence
<400> 6
   Met Asn His Lys Val His Met Glu Leu
   1 5
<210> 7
   <211> 28
   <212> DNA
   <213> artificial
   <220>
   <223> primer 1
<400> 7
   caccatgaaa ttaaaaggta ttttattt
   28
<210> 8
   <211> 23
   <212> DNA
   <213> artificial
   <220>
   <223> primer 2
<400> 8
   tactaatgta ccccacgtgt tgt
   23
<210> 9
   <211> 34
   <212> DNA
   <213> artificial
   <220>
   <223> primer 3
<400> 9
   taagagctca tgaaattaaa aggtatttta tttg
   34
<210> 10
   <211> 50
   <212> DNA
   <213> artificial
   <220>
   <223> primer 4
<400> 10
   acatctagat taatgatgat gatgatgatg tactaatgta ccccacgtgt
   50
<210> 11
   <211> 36
   <212> DNA
   <213> artificial
   <220>
   <223> primer 5
<400> 11
   ttgagctcat gcaaacagct aacgcatatg aagtta
   36
<210> 12
   <211> 50
   <212> DNA
   <213> artificial
   <220>
   <223> primer 6
<400> 12

### Literature:

Arias CA, Murray BE. 2012. The rise of the Enterococcus: beyond vancomycin resistance. Nat Rev Microbiol. 10: 266-278.
Billot-Klein D, Shlaes D, Bryant D, Bell D, van Heijenoort J, Gutmann L. 1996. Peptidoglycan structure of Enterococcus faecium expressing vancomycin resistance of the VanB type. Biochem J. 313: 711-715.
Guzman, L.-M., Belin, D., Carson, M. J., and Beckwith, J. 1995. Tight regulation, modulation, and high-level expression by vectors containing the arabinose pBAD promoter. J. Bacteriol. 177: 4121-4130.
Leclerc, D., A. Asselin. 1989. Detection of bacterial cell wall hydrolysis after denaturing polyacrylamide gel electrophoresis. Can. J. Microbiol. 35: 749-753.
Nelson D., Schmelcher M., Rodriguez-Rubio L., Klumpp J., Pritchard D. G., Dong S., Donovan D. M. 2012. Endolysins as antimicrobials. Adv. Virus. Res. 83: 299 - 365.
Qing G, Ma LC, Khorchid A, Swapna GV, Mal TK, Takayama MM, Xia B, Phadtare S, Ke H, Acton T, Montelione GT, Ikura M, Inouye M. 2004. Cold-shock induced high-yield protein production in Escherichia coli. Nat Biotechnol. 22: 877-82.
Pastagia M, Schuch R, Fischetti VA, Huang DB. 2013. Lysins: the arrival of pathogen-directed anti-infectives. J Med Microbiol. 62: 1506-1516.
Pfeffer JM, Strating H, Weadge JT, Clarke AJ. 2006. Peptidoglycan O acetylation and autolysin profile of Enterococcus faecalis in the viable but nonculturable state. J. Bacteriol. 188: 902-908.
Salazar O, Asenjo JA. 2007. Enzymatic lysis of microbial cells. Biotechnol Lett. 29: 985-994.
Sambrook et al., 1989. Molecular cloning: A Laboratory Manual, 2nd edition, Cold Spring Harbor Laboratory Press, Cold Spring Harbor, New York.
Silhavy TJ, Kahne D, Walker S. 2010. The bacterial cell envelope. Cold Spring Harb Perspect Biol. 2(5):a000414.
Son JS, Jun SY, Kim EB, Park JE, Paik HR, Yoon SJ, Kang SH, Choi YJ. 2010. Complete genome sequence of a newly isolated lytic bacteriophage, EFAP-1 of Enterococcus faecalis, and antibacterial activity of its endolysin EFAL-1. J Appl Microbiol. 108: 1769-1779.
Sugahara K, Yokoi KJ, Nakamura Y, Nishino T, Yamakawa A, Taketo A, Kodaira K. 2007. Mutational and biochemical analyses of the endolysin Lys(gaY) encoded by the Lactobacillus gasseri JCM 1131T phage phi gaY. Gene. 404: 41-52.
Uchiyama J, Takemura I, Hayashi I, Matsuzaki S, Satoh M, Ujihara T, Murakami M, Imajoh M, Sugai M, Daibata M. 2011. Characterization of lytic enzyme open reading frame 9 (ORF9) derived from Enterococcus faecalis bacteriophage phiEF24C. Appl Environ Microbiol. 77: 580-585.
Vollmer W, Blanot D, de Pedro MA. 2008. Peptidoglycan structure and architecture. FEMS Microbiol Rev. 32: 149-167.
Yoong P, Schuch R, Nelson D, Fischetti VA. 2004. Identification of a broadly active phage lytic enzyme with lethal activity against antibiotic-resistant Enterococcus faecalis and Enterococcus faecium. J Bacteriol. 186: 4808-4812.
Young R. 2005. Phage lysis. W: Phages. Their Role in Bacterial Pathogenesis and Biotechnology. Waldor K.M., Friedman D.I., Adhya S.A. (ed.) ASM Press, Washington D.C., 2005; 92-128.
Young R. 2014 Phage lysis: three steps, three choices, one outcome. J Microbiol. 52: 243 - 258.
Zhang W, Mi Z, Yin X, Fan H, An X, Zhang Z, Chen J, Tong Y. 2013. Characterization of Enterococcus faecalis phage IME-EF1 and its endolysin. PLoS One. 13;8(11):e80435.

### SEQUENCE LISTING

<110> IBB PAN
   IITD PAN
<120> Polipeptyd, koduj¹cy go polinukleotyd oraz jego zastosowania
<130> PK/2685/RW
<160> 12
<170> PatentIn version 3.5
<210> 1
   <211> 1098
   <212> DNA
   <213> Bacteriophage Enterococcus faecalis Ef12c29-ami
<400> 1
   atgaaattaa aaggtatttt atttggtgca ttagcaacca ttggtttgtt tgctggaatg      60
   caaacagcta acgcatatga agttaataac gagttcaatt taagcccttg ggaaggttca     120
   ggacaggttg cagtacctaa taagattatc ttacatgaaa ctgccaatga acgtgccaca     180
   ggacgaaatg aagcaacgta catgaaaaat aactggttta atgcacatac aacagctatc     240
   attggtgacg gtggtattgt ttataagatt gcaccagaag gtaacatttc atggggtgct     300
   ggtaatgtaa acccatacgc acctattcaa attgagttgc aacatacgca tgataaagag     360
   ttattcaaaa agaactataa agcatacatt gactatacaa gggacatggg taaaaagttt     420
   ggtattccta tgacacttga ccaaggttct tctgtttggg aaaaaggtgt tatctctcat     480
   aaatgggtat cagattatgt atggggtgac cacacagacc catatggtta cttagcagaa     540
   atgggaatca gtaaagcgca acttgctaaa gacttagcta atgggctatc tggtgaatca     600
   gtaaaaccaa caccaagcaa accaaagaca ttcaaaaaag gtcaaaacgt ttacatttat     660
   aacggtcaca aatcacacaa tggaccagtg gtaccattcg tagctggtgc aagtctttgg     720
   acccaagttg gtacaattac agaagtgaaa caaggtacag tcaatccgta taagattgaa     780
   aacagtggta aatttgtaac atatgctaac gctggcgact tagaggatct taacactaag     840
   ttcccaccaa aaccaagtaa atcagttaat cagtttacaa ttggtgttga tgctattgtt     900
   ttacgtagtg gacgaccaag cgtatatgca ccagtatatg gaacatggaa acaaggtgca     960
   gtattcaagt atgatgaaat cacagttggt gacggttatg tatggattgg tggaacagac    1020
   actaatggta cacgtattta cttaccaatt ggaccaaatg acggagaccc caacaacacg    1080
   tggggtacat tagtataa                                                  1098
<210> 2
   <211> 365
   <212> PRT
   <213> Bacteriophage Enterococcus faecalis Ef12c29-ami
<400> 2
   Met Lys Leu Lys Gly Ile Leu Phe Gly Ala Leu Ala Thr Ile Gly Leu
   1 5 10 15
   Phe Ala Gly Met Gln Thr Ala Asn Ala Tyr Glu Val Asn Asn Glu Phe
   20 25 30
   Asn Leu Ser Pro Trp Glu Gly Ser Gly Gln Val Ala Val Pro Asn Lys
   35 40 45
   Ile Ile Leu His Glu Thr Ala Asn Glu Arg Ala Thr Gly Arg Asn Glu
   50 55 60
   Ala Thr Tyr Met Lys Asn Asn Trp Phe Asn Ala His Thr Thr Ala Ile
   65 70 75 80
   Ile Gly Asp Gly Gly Ile Val Tyr Lys Ile Ala Pro Glu Gly Asn Ile
   85 90 95
   Ser Trp Gly Ala Gly Asn Val Asn Pro Tyr Ala Pro Ile Gln Ile Glu
   100 105 110
   Leu Gln His Thr His Asp Lys Glu Leu Phe Lys Lys Asn Tyr Lys Ala
   115 120 125
   Tyr Ile Asp Tyr Thr Arg Asp Met Gly Lys Lys Phe Gly Ile Pro Met
   130 135 140
   Thr Leu Asp Gln Gly Ser Ser Val Trp Glu Lys Gly Val Ile Ser His
   145 150 155 160
   Lys Trp Val Ser Asp Tyr Val Trp Gly Asp His Thr Asp Pro Tyr Gly
   165 170 175
   Tyr Leu Ala Glu Met Gly Ile Ser Lys Ala Gln Leu Ala Lys Asp Leu
   180 185 190
   Ala Asn Gly Leu Ser Gly Glu Ser Val Lys Pro Thr Pro Ser Lys Pro
   195 200 205
   Lys Thr Phe Lys Lys Gly Gln Asn Val Tyr Ile Tyr Asn Gly His Lys
   210 215 220
   Ser His Asn Gly Pro Val Val Pro Phe Val Ala Gly Ala Ser Leu Trp
   225 230 235 240
   Thr Gln Val Gly Thr Ile Thr Glu Val Lys Gln Gly Thr Val Asn Pro
                   245                 250                 255
   Tyr Lys Ile Glu Asn Ser Gly Lys Phe Val Thr Tyr Ala Asn Ala Gly
               260                 265                 270
   Asp Leu Glu Asp Leu Asn Thr Lys Phe Pro Pro Lys Pro Ser Lys Ser
           275                 280                 285
   Val Asn Gln Phe Thr Ile Gly Val Asp Ala Ile Val Leu Arg Ser Gly
       290                 295                 300
   Arg Pro Ser Val Tyr Ala Pro Val Tyr Gly Thr Trp Lys Gln Gly Ala
   305                 310                 315                 320
   Val Phe Lys Tyr Asp Glu Ile Thr Val Gly Asp Gly Tyr Val Trp Ile
                   325                 330                 335
   Gly Gly Thr Asp Thr Asn Gly Thr Arg Ile Tyr Leu Pro Ile Gly Pro
               340                 345                 350
   Asn Asp Gly Asp Pro Asn Asn Thr Trp Gly Thr Leu Val
           355                 360                 365
<210> 3
   <211> 346
   <212> PRT
   <213> artificial
<220>
   <223> protein Ef12c29-amiv1
<400> 3
   Met Gln Thr Ala Asn Ala Tyr Glu Val Asn Asn Glu Phe Asn Leu Ser
   1               5                   10                  15
   Pro Trp Glu Gly Ser Gly Gln Val Ala Val Pro Asn Lys Ile Ile Leu
               20                  25                  30
   His Glu Thr Ala Asn Glu Arg Ala Thr Gly Arg Asn Glu Ala Thr Tyr
           35                  40                  45
   Met Lys Asn Asn Trp Phe Asn Ala His Thr Thr Ala Ile Ile Gly Asp
       50                  55                  60
   Gly Gly Ile Val Tyr Lys Ile Ala Pro Glu Gly Asn Ile Ser Trp Gly
   65                  70                  75                  80
   Ala Gly Asn Val Asn Pro Tyr Ala Pro Ile Gln Ile Glu Leu Gln His
                   85                  90                  95
   Thr His Asp Lys Glu Leu Phe Lys Lys Asn Tyr Lys Ala Tyr Ile Asp
               100                 105                 110
   Tyr Thr Arg Asp Met Gly Lys Lys Phe Gly Ile Pro Met Thr Leu Asp
           115                 120                 125
   Gln Gly Ser Ser Val Trp Glu Lys Gly Val Ile Ser His Lys Trp Val
       130                 135                 140
   Ser Asp Tyr Val Trp Gly Asp His Thr Asp Pro Tyr Gly Tyr Leu Ala
   145                 150                 155                 160
   Glu Met Gly Ile Ser Lys Ala Gln Leu Ala Lys Asp Leu Ala Asn Gly
                   165                 170                 175
   Leu Ser Gly Glu Ser Val Lys Pro Thr Pro Ser Lys Pro Lys Thr Phe
               180                 185                 190
   Lys Lys Gly Gln Asn Val Tyr Ile Tyr Asn Gly His Lys Ser His Asn
           195                 200                 205
   Gly Pro Val Val Pro Phe Val Ala Gly Ala Ser Leu Trp Thr Gln Val
       210                 215                 220
   Gly Thr Ile Thr Glu Val Lys Gln Gly Thr Val Asn Pro Tyr Lys Ile
   225                 230                 235                 240
   Glu Asn Ser Gly Lys Phe Val Thr Tyr Ala Asn Ala Gly Asp Leu Glu
                   245                 250                 255
   Asp Leu Asn Thr Lys Phe Pro Pro Lys Pro Ser Lys Ser Val Asn Gln
               260                 265                 270
   Phe Thr Ile Gly Val Asp Ala Ile Val Leu Arg Ser Gly Arg Pro Ser
           275                 280                 285
   Val Tyr Ala Pro Val Tyr Gly Thr Trp Lys Gln Gly Ala Val Phe Lys
       290                 295                 300
   Tyr Asp Glu Ile Thr Val Gly Asp Gly Tyr Val Trp Ile Gly Gly Thr
   305                 310                 315                 320
   Asp Thr Asn Gly Thr Arg Ile Tyr Leu Pro Ile Gly Pro Asn Asp Gly
                   325                 330                 335
   Asp Pro Asn Asn Thr Trp Gly Thr Leu Val
   340 345
<210> 4
   <211> 355
   <212> PRT
   <213> artificial
<220>
   <223> protein Ef12c29-amiv2
<400> 4
   Met Asn His Lys Val His Met Glu Leu Met Gln Thr Ala Asn Ala Tyr
   1               5                   10                  15
   Glu Val Asn Asn Glu Phe Asn Leu Ser Pro Trp Glu Gly Ser Gly Gln
               20                  25                  30
   Val Ala Val Pro Asn Lys Ile Ile Leu His Glu Thr Ala Asn Glu Arg
           35                  40                  45
   Ala Thr Gly Arg Asn Glu Ala Thr Tyr Met Lys Asn Asn Trp Phe Asn
       50                  55                  60
   Ala His Thr Thr Ala Ile Ile Gly Asp Gly Gly Ile Val Tyr Lys Ile
   65                  70                  75                  80
   Ala Pro Glu Gly Asn Ile Ser Trp Gly Ala Gly Asn Val Asn Pro Tyr
                   85                  90                  95
   Ala Pro Ile Gln Ile Glu Leu Gln His Thr His Asp Lys Glu Leu Phe
               100                 105                 110
   Lys Lys Asn Tyr Lys Ala Tyr Ile Asp Tyr Thr Arg Asp Met Gly Lys
           115                 120                 125
   Lys Phe Gly Ile Pro Met Thr Leu Asp Gln Gly Ser Ser Val Trp Glu
       130                 135                 140
   Lys Gly Val Ile Ser His Lys Trp Val Ser Asp Tyr Val Trp Gly Asp
   145                 150                 155                 160
   His Thr Asp Pro Tyr Gly Tyr Leu Ala Glu Met Gly Ile Ser Lys Ala
                   165                 170                 175
   Gln Leu Ala Lys Asp Leu Ala Asn Gly Leu Ser Gly Glu Ser Val Lys
               180                 185                 190
   Pro Thr Pro Ser Lys Pro Lys Thr Phe Lys Lys Gly Gln Asn Val Tyr
           195                 200                 205
   Ile Tyr Asn Gly His Lys Ser His Asn Gly Pro Val Val Pro Phe Val
       210                 215                 220
   Ala Gly Ala Ser Leu Trp Thr Gln Val Gly Thr Ile Thr Glu Val Lys
   225                 230                 235                 240
   Gln Gly Thr Val Asn Pro Tyr Lys Ile Glu Asn Ser Gly Lys Phe Val
                   245                 250                 255
   Thr Tyr Ala Asn Ala Gly Asp Leu Glu Asp Leu Asn Thr Lys Phe Pro
               260                 265                 270
   Pro Lys Pro Ser Lys Ser Val Asn Gln Phe Thr Ile Gly Val Asp Ala
           275                 280                 285
   Ile Val Leu Arg Ser Gly Arg Pro Ser Val Tyr Ala Pro Val Tyr Gly
       290                 295                 300
   Thr Trp Lys Gln Gly Ala Val Phe Lys Tyr Asp Glu Ile Thr Val Gly
   305                 310                 315                 320
   Asp Gly Tyr Val Trp Ile Gly Gly Thr Asp Thr Asn Gly Thr Arg Ile
                   325                 330                 335
   Tyr Leu Pro Ile Gly Pro Asn Asp Gly Asp Pro Asn Asn Thr Trp Gly
               340                 345                 350
   Thr Leu Val
           355
<210> 5
   <211> 27
   <212> DNA
   <213> artificial
<220>
   <223> alternative 5' sequence
<400> 5
   atgaatcaca aagtgcatat ggagctc 27
<210> 6
   <211> 9
   <212> PRT
   <213> artificial
<220>
   <223> alternative N-sequence
<400> 6
   Met Asn His Lys Val His Met Glu Leu
   1 5
<210> 7
   <211> 28
   <212> DNA
   <213> artificial
<220>
   <223> primer 1
<400> 7
   caccatgaaa ttaaaaggta ttttattt 28
<210> 8
   <211> 23
   <212> DNA
   <213> artificial
<220>
   <223> primer 2
<400> 8
   tactaatgta ccccacgtgt tgt 23
<210> 9
   <211> 34
   <212> DNA
   <213> artificial
<220>
   <223> primer 3
<400> 9
   taagagctca tgaaattaaa aggtatttta tttg 34
<210> 10
   <211> 50
   <212> DNA
   <213> artificial
<220>
   <223> primer 4
<400> 10
   acatctagat taatgatgat gatgatgatg tactaatgta ccccacgtgt 50
<210> 11
   <211> 36
   <212> DNA
   <213> artificial
<220>
   <223> primer 5
<400> 11
   ttgagctcat gcaaacagct aacgcatatg aagtta 36
<210> 12
   <211> 50
   <212> DNA
   <213> artificial
<220>
   <223> primer 6
<400> 12
   acatctagat taatgatgat gatgatgatg tactaatgta ccccacgtgt 50

## Claims

1. A polypeptide having the activity of digestion of peptidoglycan, particularly one produced by bacterial cells of the genus *Enterococcus,* comprising the amino acid sequence designated as SEQ ID NO: 3.

2. The polypeptide according to claim 1, **characterized in that** it comprises an amino acid sequence designated as SEQ ID NO: 4.

3. A polynucleotide comprising a sequence encoding the polypeptide according to claim 1 or 2.

4. The polynucleotide according to claim 3, **characterized in that** it contains the nucleotide sequence designated as SEQ ID NO: 1.

5. Use of the polypeptide according to claim 1 or 2 for non-therapeutic digestion of peptidoglycan, particularly one produced by bacterial cells of the genus *Enterococcus.*

6. The polypeptide according to claim 1 or 2, for use in the treatment or prevention of bacterial infections, particularly those caused by bacteria of the genus *Enterococcus.*

## Patentansprüche

1. Polypeptid das eine Peptidoglykan-verdauende Aktivität aufweist, insbesondere eines, das durch bakterielle Zellen des Genus Enterococcus hergestellt wird, umfassend die als SEQ ID NO: 3 bezeichnete Aminosäuresequenz.

2. Polypeptid nach Anspruch 1, **dadurch gekennzeichnet, dass** es eine als SEQ ID NO: 4 bezeichnete Aminosäuresequenz umfasst.

3. Polynukleotid, umfassend eine Sequenz, die für das Polypeptid nach Anspruch 1 oder 2 kodiert.

4. Polynukleotid nach Anspruch 3, **dadurch gekennzeichnet, dass** es die als SEQ ID NO: 1 bezeichnete Nukcleotidsequenz enthält.

5. Verwendung des Polypeptids nach Anspruch 1 oder 2 zum nicht-therapeutischen Verdau von Peptidoglykan, insbesondere eines, das durch bakterielle Zellen des Genus Enterococcus hergestellt wird.

6. Polypeptid nach Anspruch 1 oder 2 zur Verwendung in der Behandlung oder der Vorbeugung von bakteriellen Infektionen, insbesondere denjenigen, die durch Bakterien des Genus Enterococcus verursacht werden.

## Revendications

1. Polypeptide ayant l'activité de digestion de peptidoglycane, particulièrement un produit par des cellules bactériennes du genre *Enterococcus,* comprenant la séquence d'acides aminés désignée par SEQ ID NO: 3.

2. Polypeptide selon la revendication 1, **caractérisé en ce qu'**il comprend une séquence d'acides aminés désignée par SEQ ID NO: 4.

3. Polynucléotide comprenant une séquence codant le polypeptide selon la revendication 1 ou 2.

4. Polynucléotide selon la revendication 3, **caractérisé en ce qu'**il contient la séquence de nucléotides désignée par SEQ ID NO: 1.

5. Utilisation du polypeptide selon la revendication 1 ou 2 pour la digestion non thérapeutique de peptidoglycane, particulièrement d'un produit par des cellules bactériennes du genre *Enterococcus.*

6. Polypeptide selon la revendication 1 ou 2, pour utilisation dans le traitement ou la prévention d'infections bactériennes, particulièrement celles provoquées par des bactéries du genre *Enterococcus.*
